# EUROPEAN PATENT APPLICATION

(11) **EP 3 985 390 A1**
(43) Date of publication of application: **20.04.2022**
(21) Application number: 20825796.4
(22) Date of filing: 12.06.2020
(51) Int. Cl.: G01N 30/72, G01N 30/88

(54) **BIOMARKER FOR PREDICTING OR CLASSIFYING SEVERITY OF RHEUMATOID ARTHRITIS USING METABOLITE ANALYSIS**

(30) Priority: 17.06.2019 KR 20190071462
(71) Applicant: Korea University Research and Business Foundation, Seoul 02841 (KR)
(72) Inventor: KIM, Kyoung Heon, Seoul 06601 (KR); CHA, Hoon Suk, Seoul 06351 (KR); AHN, Joong Kyoung, Seoul 03181 (KR); KIM, Jung Yeon, Seoul 02412 (KR); CHEONG, Yu Eun, Suwon-si Gyeonggi-do 16509 (KR)
(74) Representative: Plasseraud IP
(86) International application number: PCT/KR2020/007646
(87) International publication number: WO 2020/256351

(57) **Abstract**

The present invention relates to a method for diagnosing the severity of rheumatoid arthritis using metabolite analysis. The present invention provides a biomarker which is for classifying the severity of rheumatoid arthritis in patients suffering from same through the analysis of joint capsule fluid metabolites, and can be applied to a kit for more specifically classifying the severity of rheumatoid arthritis in patients.

## Description

### [Technical Field]

The present invention relates to metabolite biomarkers for predicting or classifying the severity of rheumatoid arthritis using the analysis of a synovial fluid metabolite.

### [Background Art]

Rheumatoid arthritis (RA) is a representative chronic disease which occurs due to inflammation in tissue such as the synovial membrane surrounding the joint, and is estimated to affect 1% of the total population in Korea (McInnes I. B. and Schett G. The pathogenesis of rheumatoid arthritis (2011) N Engl J Med vol. 365, pp. 2205-2219). Synovial fluid does not only act as a biological lubricant in joints, but also acts as a fluid through which nutrients and various cytokines pass. Therefore, inflammation in the joint synovial membrane and cartilage or enzyme-mediated degradation causes a change in chemical composition of the synovial fluid. In addition, the synovial fluid is considered as a sample that best reflects the etiological condition of inflammatory arthritis (O'Connel J. X. Pathology of the synovium (2000) Am J Clin Pathol vol. 114, pp. 773-784).

Therefore, the analysis of synovial fluid in a rheumatoid arthritis patient may be applied to clinical practice by providing a biomarker for diagnosis, or helping to understand the etiology of rheumatoid arthritis.

In addition, as a therapeutic method for rheumatoid arthritis, in order to minimize joint damage, prevent loss of function and reduce pain, a drug treatment method in which an analgesic/anti-inflammatory is administered in combination with various anti-rheumatoid drugs is generally used; a biotherapeutic agent has been recently developed, and is being used in combination therapy with an anti-rheumatoid drug; and when a disease has high severity, surgery is performed. However, despite a very excellent effect, such a treatment method may have persistent joint deformation and difficulty in appropriate treatment due to drug side effects. Moreover, since there is a disadvantage of incurring a high cost due to an increase in drug price according to the cost of developing a new drug, there is an urgent need to develop a kit capable of predicting or classifying the severity of rheumatoid arthritis.

Metabolomics is a study that examines a change in overall metabolites according to a metabolic change in the body, and is used to identify various physiological and pathological conditions (Johnson C. H. et. al. Metabolomics: beyond biomarkers and towards mechanisms (2016) Nat Rev Mol Cell Biol vol. 17, pp. 451-459). Since RA is caused by the action of various factors including genetic and environmental factors, a change in metabolic material may be induced, and metabolomics may be useful for revealing physiological and pathological changes.

A biomarker capable of differentiating between other arthritis patients and a rheumatoid arthritis patient has been reported in the non-patent literature (Kim S. et. al. Global metabolite profiling of synovial fluid for the specific diagnosis of rheumatoid arthritis from other inflammatory arthritis (2014) PLOS ONE vol. 9(6), e97501), but the present invention is characterized in that it relates to a biomarker for predicting or classifying the severity of a RA patient.

### [Disclosure]

### [Technical Problem]

The inventors examined how a metabolic profile changes according to severity in the synovial fluid of a rheumatoid arthritis patient through metabolomics, suggested a metabolite marker indicating severity, and discovered a novel biomarker capable of more specifically classifying the severity of patients.

The synovial fluid of a rheumatoid arthritis patient was collected, and a total of 125 metabolites were detected by the analysis of metabolites in the synovial fluid of the rheumatoid arthritis patient by GC/TOF MS. Based on this, the analysis was correlated with the patient's severity level (DAS28-ESR).

First, the severity of each patient was calculated by DAS28-ESR, and metabolites that statistically significantly increase or decrease according to the increase in severity were found by obtaining the Spearman's rank correlation coefficient, which is a non-parametric correlation analysis. Here, 14 metabolites with a p-value of less than 0.05 were selected as candidates for novel biomarkers.

A multivariate statistical method-based orthogonal partial least squares discriminant analysis (OPLS-DA) model was made using the 14 candidates for novel biomarkers indicating the severity of the rheumatoid arthritis patients, thereby making it possible to distinguish between a high disease activity group and a moderate disease activity group.

In addition, to confirm whether a severity diagnostic model can be also applied to diagnose the severity of an external sample, it was confirmed that the severity can be exactly determined by analyzing metabolites in synovial fluid samples obtained from 10 patients and applying them to the model, and the model was verified.

Therefore, the present invention is directed to providing a kit for classifying rheumatoid arthritis patients into a high disease activity group and a moderate disease activity group.

The present invention is also directed to providing a kit for predicting the severity of a rheumatoid arthritis patient.

### [Technical Solution]

The present invention provides a kit for classifying rheumatoid arthritis patients into a high disease activity group and a moderate disease activity group, which includes a quantification device for one or more synovial fluid metabolites selected from the group consisting of indole-3-lactate, glucose-6-phosphate, fucose, isothreonate, 3-phenyllactate, guaiacol, glycocyamine, adipate, phenylalanine, arabitol, cholic acid and tryptophan.

In addition, the present invention provides a kit for predicting the severity of a rheumatoid arthritis patient, which includes a quantification device for one or more synovial fluid metabolites selected from the group consisting of indole-3-lactate, glucose-6-phosphate, fucose, isothreonate, 3-phenyllactate, guaiacol, glycocyamine, adipate, phenylalanine, arabitol, cholic acid and tryptophan.

### [Advantageous Effects]

Through the present invention, a biomarker that can specifically predict or classify the severity of rheumatoid arthritis using metabolomics was identified. Such a biomarker for predicting or classifying severity can be applied in various forms, such as a severity classification kit for predicting severity or classifying into high disease activity and moderate disease activity.

Therefore, the present invention can be applied in novel drug development targeting a biomarker specific for the severity of rheumatoid arthritis, and by using a biomarker specific for the severity of rheumatoid arthritis, the pathogenesis of rheumatoid arthritis can be more accurately identified, and based on this, can be used in novel drug development and as a tool for screening novel drug candidates.

In addition, since customized early treatment is possible according to classification and prediction of rheumatoid arthritis, severity can be determined with the concentration of a specific metabolite for a patient suspected of or diagnosed with rheumatoid arthritis so it can be applied in customized early treatment for rheumatoid arthritis.

### [Description of Drawings]

FIG. 1 shows diagnostic models (a: score plot; b: loading plot; c: permutation tests) by comparing rheumatoid arthritis patients between a high disease activity group (RA_high) and a moderate disease activity group (RA_moderate) using OPLS-DA generated based on 14 potential biomarkers for distinguishing severity.
FIG. 2 shows statistical verification on an OPLS-DA model for diagnosing the severity of rheumatoid arthritis patients using an ROC curve.
FIG. 3 shows severity diagnosis verification of an OPLS-DA model for diagnosing the severity of rheumatoid arthritis patients using foreign specimens.

### [Modes of the Invention]

Hereinafter, the present invention will be described in further detail.

The present invention relates to a kit for predicting the severity of rheumatoid arthritis patients and/or a kit for classifying rheumatoid arthritis patients into a high disease activity group and a moderate disease activity group.

The term "prediction of the prognosis of the severity of rheumatoid arthritis" used herein is associated with the possibility of bone destruction or joint deformation due to high disease activity when the overall degree of inflammation, bone destruction in rheumatoid arthritis patients, or the progression of rheumatoid arthritis was evaluated. Disease Activity Score (DAS) or its modified form Disease Activity Score 28 (DAS28), which are representative methods for measuring disease severity of rheumatoid arthritis in conventional clinical practices, are score calculation-based index assessment methods, and assess subjective factors evaluated by patients and doctors along with objective factors such as inflammation marker tests or radiographic findings. DAS28 is a comprehensive index consisting of the number of joints with tenderness and the number of joints with swelling among 28 joints of a patient, an erythrocyte sedimentation rate and a patient's systemic evaluation, and the 28 joints include both shoulder joints, elbow and wrist joints, metacarpophalangeal joints, proximal interphalangeal joints and knee joints.

The discrimination of the severity of a disease using DAS28 is performed by a DAS28-ESR score based on an erythrocyte sedimentation rate and a DAS28-CRP score calculated based on the activity index of a C-reactive protein, and particularly, a DAS28-ESR(3) score calculating the severity of a disease using a DAS28-ESR score, a tender joint count and a swollen joint count, omitting other comprehensive examinations on a patient, is widely used (https://www.mdcalc.com/disease-activity-score-28-rheumatoid-arthritis-esr-das28-esr).

The rheumatoid arthritis disease activity investigated by the DAS28-ESR(3) score may be calculated from 0 to a maximum of 9.4, and generally, when the DAS28 score is less than 2.6, it is defined as remission, when the score is 2.6 or more and less than 3.2, it is defined as low disease activity (mild), when the score is 3.2 or more and less than 5.1, it is defined as moderate disease activity (moderate), and when the score is 5.1 or more, it is defined as high disease activity (high).

The present invention can easily predict the future severity of a disease of a patient, and it may be decided to use an additionally required therapeutic method so it can be used as information for delaying, alleviating and/or curing a disease after the onset of rheumatoid arthritis.

According to one embodiment of the present invention, a metabolite sampling step including extracting a metabolite by mixing pure methanol with synovial fluid, strongly vortexing the mixture and then performing centrifugation is included.

As a solvent used for extraction of the metabolite, methanol may be used, but the present invention is not particularly limited thereto.

The 125 metabolites include amines, amino acids, sugars, sugar alcohols, fatty acids, phosphates and organic acids.

The metabolite extracted in the metabolite sampling step undergoes the following analysis steps:
analyzing the extracted metabolite using a gas chromatography/time-of-flight mass spectrometry (GC/TOF) instrument;
converting the GC/TOF MS analysis result into a statistically processable value; and
statistically verifying the distinction between the two biological sample groups using the converted value.

That is, in the step of converting the GC/TOF MS analysis result into a statistically processable value, the total analysis time is divided by a unit time interval, and the largest value of the areas or heights of chromatogram peaks shown during the unit time is determined as a representative value during the unit time.

Subsequently, to compare the profiling difference between metabolites, partial least squares discriminant analysis (PLS-DA) is performed to select, analyze and verify a metabolite biomarker showing a significant difference between two biological sample groups.

A positive loading value of PLS-DA is determined as an increasing tendency of a metabolite biomarker, and a negative loading value thereof is determined as a decreasing tendency of a metabolite biomarker.

According to one embodiment of the present invention, as a biomarker for distinguishing metabolites of a high rheumatoid arthritis activity group and a moderate rheumatoid arthritis activity group, one or more metabolites selected from the group consisting of indole-3-lactate, glucose-6-phosphate, fucose, isothreonate, 3-phenyllactate, guaiacol, glycocyamine, adipate, phenylalanine, arabitol, cholic acid and tryptophan may be used.

In the high disease activity group, among the biomarkers, one or more metabolites selected from the group consisting of indole-3-lactate, glucose-6-phosphate, fucose, isothreonate, 3-phenyllactate, guaiacol, glycocyamine, adipate, phenylalanine, arabitol, cholic acid and tryptophan show an increasing tendency, and citrate and asparagine show a decreasing tendency.

On the other hand, in the moderate disease activity group, among the biomarkers, one or more metabolites selected from the group consisting of indole-3-lactate, glucose-6-phosphate, fucose, isothreonate, 3-phenyllactate, guaiacol, glycocyamine, adipate, phenylalanine, arabitol, cholic acid and tryptophan show a decreasing tendency, and citrate and asparagine show an increasing tendency.

The increasing or decreasing tendency means an increase or decrease in metabolite concentration, and the term "increase in metabolite concentration" means that the metabolite concentration in the high disease activity group of rheumatoid arthritis patients, compared to the moderate disease activity group, or in the moderate disease activity group of rheumatoid arthritis patients, compared to the high disease activity is significantly increased to a measurable degree, and the term "decrease in metabolite concentration" used herein means that the metabolite concentration is significantly decreased in the high disease activity group of rheumatoid arthritis patients, compared to the moderate disease activity group thereof, or in the moderate disease activity group of rheumatoid arthritis patients, compared to the high disease activity group thereof.

Therefore, the present invention provides a kit for classifying rheumatoid arthritis patients into a high disease activity group and a moderate disease activity group, which includes a quantification device for one or more metabolites selected from the group consisting of indole-3-lactate, citrate, glucose-6-phosphate, fucose, isothreonate, 3-phenyllactate, guaiacol, glycocyamine, adipate, phenylalanine, arabitol, asparagine, cholic acid, and tryptophan

Subsequently, to find a metabolite which significantly increases/decreases depending on an increase in the severity of rheumatoid arthritis, a metabolite biomarker showing a statistically significant increase/decrease is selected, analyzed and verified by analysis using Spearman's rank correlation coefficient (Spearman R), which is a nonparametric statistical analysis method for correlation between two variables for obtaining the correlation between the DAS-28 ESR (3) score and the intensity of a metabolite obtained in Example 1.

A positive Spearman R value shows the tendency of increasing the intensity of a metabolite biomarker according to the increase in disease severity, and a negative Spearman R value shows the tendency of decreasing the intensity of a metabolite biomarker according to the increase in disease severity.

According to one embodiment of the present invention, as a biomarker for predicting the severity of rheumatoid arthritis, one or more metabolites selected from the group consisting of indole-3-lactate, citrate, glucose-6-phosphate, fucose, isothreonate, 3-phenyllactate, guaiacol, glycocyamine, adipate, phenylalanine, arabitol, asparagine, cholic acid and tryptophan may be used.

Among the biomarkers, one or more selected from the group consisting of citrate and asparagine show a decreasing tendency according to an increase in severity, and one or more selected from the group consisting of indole-3-lactate, citrate, glucose-6-phosphate, fucose, isothreonate, 3-phenyllactate, guaiacol, glycocyamine, adipate, phenylalanine, arabitol, asparagine, cholic acid and tryptophan show an increasing tendency.

The increasing or decreasing tendency means an increase or decrease in metabolite concentration, the term "increase in metabolite concentration" means that the metabolite concentration is significantly increased to a measurable level according to the increase in the severity of a rheumatoid arthritis patient, and the term "decrease in metabolite concentration" used herein means that the metabolite concentration is significantly decreased to a measurable level according to the increase in the severity of a rheumatoid arthritis patient.

The quantification device included in the kit of the present invention may be an instrument for chromatography/mass spectrometry. Specifically, gas chromatography, liquid-solid chromatography (LSC), paper chromatography (PC), thin-layer chromatography (TLC), gas-solid chromatography (GSC), liquid-liquid chromatography (LLC), foam chromatography (FC), emulsion chromatography (EC), gas-liquid chromatography (GLC), ion chromatography (IC), gel filtration chromatography (GFC) or gel permeation chromatography (GPC) may be used, but the present invention is not limited thereto, and thus any chromatography for quantification, which is conventionally used in the art, may be used. More specifically, the instrument may be an instrument for gas chromatography/time-of-flight mass spectrometry (GC/TOF MS).

Each component of the metabolite of the present invention is isolated by gas chromatography, and using information obtained by TOF MS, the components are identified not only through exact molecular weight data but also elemental composition.

Hereinafter, the present invention will be described in further detail with reference to examples according to the present invention, but the scope of the present invention is not limited by the following examples .

### [EXAMPLES]

### Example 1: Identification of metabolite of synovial fluid from rheumatoid arthritis patient using GC/TOF MS

Synovial fluids were collected from 30 rheumatoid arthritis patients, 900 µl of pure methanol was added to 100 µl of each synovial fluid sample and strongly vortexed, and then metabolites were extracted from 40 different samples by centrifugation.

A derivatization process for GC/TOF MS analysis is as follows.

After drying the extracted sample with a speed bag, 5 µl of 40%(w/v) O-methylhydroxylamine hydrochloride in pyridine was added, and reacted at 30 °C and 200 rpm for 90 minutes. In addition, 45 µl of *N*-methyl-*N-*(trimethylsilyl)trifluoroacetamide was added, and reacted at 37 °C and 200 rpm for 30 minutes.

Conditions for an instrument for GC/TOF MS analysis are as follows.

A column used in analysis was an RTX-5Sil MS capillary column (length: 30 m, film thickness: 0.25 mm, and inner diameter: 25 mm), and a GC column temperature condition included maintenance at 50 °C for 5 minutes, an increase in temperature to 330 °C, and then maintenance for 1 minute. 1 µL of a sample was injected into GC in splitless mode. A transfer line temperature and an ion source temperature were maintained at 280 °C and 250 °C, respectively. 125 metabolites were identified from a library containing GC/TOF MS results (Table 1).

As shown in Table 1 below, the metabolites were classified by group, such as organic acids (20.8%), amino acids (21.6%), sugars (18.4%), fatty acids (14.4%), amines (11.2%), phosphates (5.6%), and miscellaneous (7.9%).

### [Table 1]

Confirmation of 125 metabolites extracted from synovial fluid of rheumatoid arthritis patients

| **Amines** | | |
|---|---|---|
| 2-hydroxypyridine | 3-hydroxypyridine | 5'-deoxy-5'-methylthioadenosine |
| glycocyamine | Guanosine | hypoxanthine |
| inosine | O-phosphorylethanolamine | thymine |
| uracil | Urea | uric acid |
| uridine | Xanthine | |

| **Amino acids** | | |
|---|---|---|
| alanine | Asparagine | asparagine dehydrated |
| aspartate | cyano- L-alanine | glutamate |
| glutamine | Glycine | histidine |
| isoleucine | L-citrulline | L-cysteine |
| leucine | L-homoserine | lysine |
| methionine | N -methylalanine | ornithine |
| oxoproline | phenylalanine | proline |
| serine | Threonine | tryptophan |
| tyrosine | Valine | β-alanine |

| **Fatty acids** | | |
|---|---|---|
| 1-monopalmitin | 1-monostearin | 2-ketoisocaproic acid |
| arachidonic acid | behenic acid | capric acid |
| heptadecanoic acid | lauric acid | lignoceric acid |
| linoleic acid | linolenic acid | myristic acid |
| oleic acid | palatinitol | palmitic acid |
| palmitoleic acid | pelargonic acid | stearic acid |

| **Organic acids** | | |
|---|---|---|
| 2-hydroxyhexanoate | 2-hydroxyvalerate | 2-ketoadipic acid |
| 3-phenyllactate | adipate | citramalate |
| citrate | DL-3-aminoisobutyrate | fumarate |
| galactonate | glycerate | glycolate |
| guaiacol | hexonate | indole-3-lactate |
| isothreonate | malate | malonate |
| oxalate | phenylacetate | pyrrole-2-carboxylate |
| pyruvate | succinate | terephthalate |
| α-ketoglutarate | β-hydroxybutyrate | |

| **Sugars and sugar alcohols** | | |
|---|---|---|
| 1,5-anhydroglucitol | 3,6-anhydro-D-galactose | arabitol |
| fructose | fucose | galactose |
| glucose | glycerol | lactose |
| lyxose | maltotriose | mannitol |
| mannose | melezitose | melibiose |
| myo-inositol | ribose | sucrose |
| threitol | threose | trehalose |
| xylose | β-gentiobiose | |

| **Phosphates** | | |
|---|---|---|
| adenosine-5-monophosphate | glucose-6-phosphate | glycerol-1-phosphate |
| mannose-6-phosphate | phosphogluconic acid | pyrophosphate |
| β-glycerolphosphate | | |

| **Miscellaneous** | | |
|---|---|---|
| 1,2,4-benzenetriol | benzoate | cholic acid |
| nicotinamide | phthalic acid | salicylaldehyde |
| salicylic acid | sulfuric acid | taurine |
| α-tocopherol | | |

### Example 2: Analysis of correlation between rheumatoid arthritis severity and metabolites using Spearman's rank correlation coefficient and suggestion of biomarker for classifying potential severity

To examine metabolic materials significantly increasing/decreasing according to an increase in severity of rheumatoid arthritis, a DAS-28 ESR (3) score indicating severity was calculated from 30 rheumatoid arthritis patients, and how this score correlated with the metabolite intensity of each patient was analyzed using Spearman's rank correlation coefficient (Table 2). The Spearman's rank correlation coefficient is a statistical method for analyzing the correlation between two different variables, and here, it was applied to examine whether the metabolite which was at a high or low level in patients with moderate disease activity was statistically significantly increased or decreased in patients with high disease activity. As a result, the correlation between the DAS-28 ESR (3) scores and the intensity of the detected 125 metabolites in the 30 patients was obtained, and among the 125 metabolites, there were 14 metabolites showing that the p-value was less than 0.05 when the DAS-28 ESR (3) score, which is the severity of a patient, increased, indicating a statistically significantly increasing or decreasing correlation (Table 2). In Table 2, N represents the number of samples, and Spearman R represents a degree of decreasing or increasing the intensity of a metabolite according to an increase in DAS-28 ESR (3) score, that is, severity. t(N-2) represents a relative value statistically calculated in correlation analysis using Spearman R, and the p-value represents the confidence interval showing how much each metabolite statistically significantly increased or decreased according to the increase in DAS-28 ESR (3) score. In the correlation analysis using Spearman R, it was suggested that the increase/decrease of metabolites according to the increase in DAS-28 ESR (3) score is statistically significant when the p-value is at a level of less than 0.05.

**[Table 2]**

| Correlation between DAS28-ESR(3) score and intensity of metabolic material of rheumatoid arthritis patient using Spearman's rank correlation coefficient | | | | |
|---|---|---|---|---|
| **Metabolite** | **N** | **Spearman R (ESR** vs. **intensity** | **t(N-2)** | ***p* value** |
| citrate | 30 | -0.58 | -3.81 | 7.01.E-04 |
| asparagine | 30 | -0.38 | -2.16 | 3.97.E-02 |
| tryptophan | 30 | 0.37 | 2.08 | 4.72.E-02 |
| cholic acid | 30 | 0.38 | 2.15 | 3.99.E-02 |
| arabitol | 30 | 0.39 | 2.22 | 3.44.E-02 |
| phenylalanine | 30 | 0.41 | 2.38 | 2.43.E-02 |
| adipate | 30 | 0.42 | 2.42 | 2.22.E-02 |
| glycocyamine | 30 | 0.43 | 2.55 | 1.63.E-02 |
| guaiacol | 30 | 0.46 | 2.72 | 1.11.E-02 |
| 3-phenyllactate | 30 | 0.48 | 2.87 | 7.68.E-03 |
| isothreonate | 30 | 0.52 | 3.19 | 3.48.E-03 |
| fucose | 30 | 0.53 | 3.30 | 2.62.E-03 |
| glucose-6-phosphate | 30 | 0.53 | 3.34 | 2.36.E-03 |
| indole-3-lactate | 30 | 0.60 | 3.96 | 4.70.E-04 |

### Example 3: Establishment of diagnostic models for severity classification based on OPLS-DA multivariate models created using 14 potential biomarkers

To establish a diagnostic model for severity classification, samples were divided into a high disease activity group and a moderate disease activity group according to the DAS28-ESR(3) score of each patient. When the DAS-ESR(3) score was 5.1 or more, the sample was classified as a high disease activity group, and when the DAS-ESR(3) score was less than 5.1, the sample was classified as a moderate disease activity group.

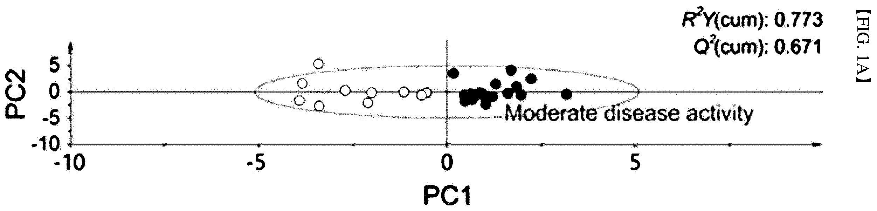

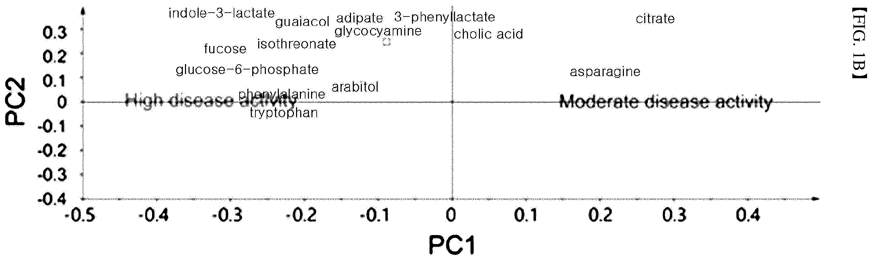

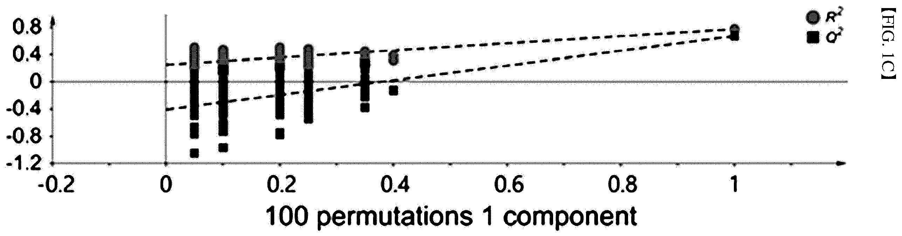

When the 14 potential metabolites for diagnosing severity suggested in Example 2 were examined through a multivariate statistics and modeling OPLS-DA technique to see whether patients with high disease activity and patients with moderate disease activity can be distinguished based on their intensity, it was confirmed that there was a clear difference between metabolite profiles (FIG. 1). FIG. 1A is a PLS-DA score plot, in which a patient of the moderate disease activity group has a positive value for PC1, and a patient of the high disease activity group has a negative value for PC2, showing that synovial fluid samples from patients were clearly classified according to the intensity of 14 metabolites except one sample from the patient with moderate disease activity. FIG. 1B is an OPLS-DA loading plot, showing the contributions of each metabolite to model formation as a positive or negative value. FIG. 1C shows the result of permutation tests for OPLS-DA models, showing that an OPLS-DA model differentiating between a high disease activity group and a moderate disease activity group is statistically significant.

### Example 4: Verification of effectiveness of OPLS-DA multivariate diagnostic model for classifying severity based on verification of external sample diagnosis

To examine whether an OPLS-DA model, which is a metabolite biomarker for diagnosing the severity of a rheumatoid arthritis patient using a synovial fluid sample prepared in Example 3, was suitable for diagnosis, a receiver operating characteristic (ROC) curve was plotted using the PC1 score of each sample in the model.

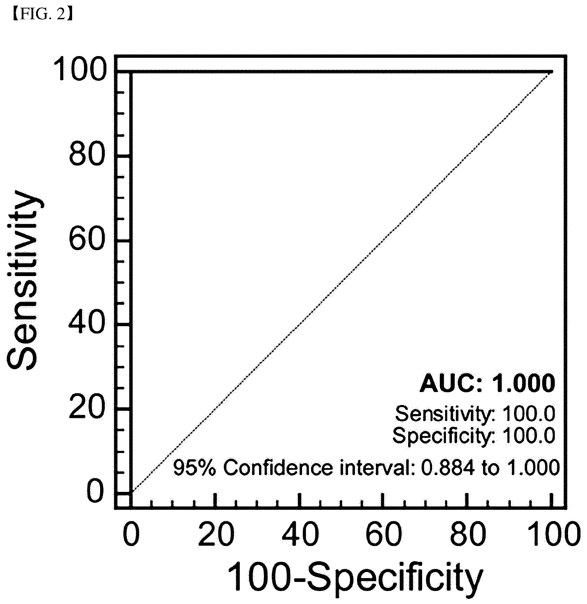

As a result, the sensitivity was 100%, the 1-specificity was 100%, and the AUC value was 1.000, showing that the model is very suitable for diagnosis of the severity of rheumatoid arthritis patients (FIG. 2).

In addition, to examine whether this model is suitable for diagnosis of the severity of rheumatoid arthritis patients using an external sample, metabolites were extracted from three synovial fluid samples from rheumatoid arthritis patients in the high disease activity group and seven synovial fluid samples from rheumatoid arthritis patients in the moderate disease activity group, and then 14 metabolites were screened by the method described in Example 1, and put into the model to examine that they can distinguish between the high disease activity group and the moderate disease activity group.

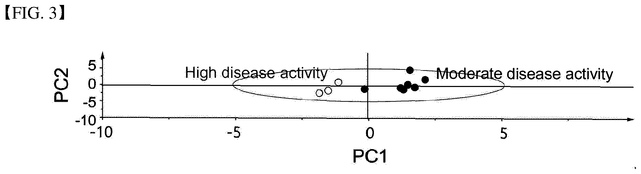

The model of FIG. 1 is an OPLS-DA model showing a negative value based on PC1 in the case of a patient with high disease activity and a positive value based on PC1 in the case of a patient with moderate disease activity when the intensity of the 14 metabolites extracted from the synovial fluid samples of the rheumatoid arthritis patients were input. Likewise, when the intensity data of the extracted 14 metabolites was input to the model, 10 external samples show negative or positive values based on PC1 according to the severity, and may be determined to have the high disease activity or moderate disease activity. As a result, among the 10 samples, three samples from the patients with high disease activity showed negative values based on PC1, and six of the 7 samples from the patients with moderate disease activity showed positive values based on PC1, and the last one showed a negative value based on PC1. Therefore, since the severity of 9 samples of a total of the 10 samples was accurately predicted, it was able to be seen that the 14-metabolite biomarkers, OPLS-DA models, are suitable for diagnosis of the severity of external samples (FIG. 3).

## Claims

1. A kit for classifying rheumatoid arthritis patients into a high disease activity group and a moderate disease activity group, comprising:
a quantification device for one or more synovial fluid metabolites selected from the group consisting of indole-3-lactate, citrate, glucose-6-phosphate, fucose, isothreonate, 3-phenyllactate, guaiacol, glycocyamine, adipate, phenylalanine, arabitol, asparagine, cholic acid, and tryptophan.

2. The kit of claim 1, wherein the quantification device is an instrument for chromatography/mass spectroscopy.

3. The kit of claim 1, wherein, when the concentration(s) of one or more selected from indole-3-lactate, glucose-6-phosphate, fucose, isothreonate, 3-phenyllactate, guaiacol, glycocyamine, adipate, phenylalanine, arabitol, cholic acid and tryptophan show(s) an increasing tendency, the patient(s) correspond(s) to the high disease activity group.

4. The kit of claim 1, wherein, when the concentration(s) of one or more selected from the group consisting of citrate and asparagine show(s) a decreasing tendency, the patient(s) correspond(s) to the high disease activity group.

5. The kit of claim 1, wherein, when the concentration(s) of one or more selected from the group consisting of citrate and asparagine show(s) increasing tendency, the patient(s) is/are assigned to the moderate disease activity group.

6. The kit of claim 1, wherein, when the concentration(s) of one or more selected from the group consisting of indole-3-lactate, glucose-6-phosphate, fucose, isothreonate, 3-phenyllactate, guaiacol, glycocyamine, adipate, phenylalanine, arabitol, cholic acid and tryptophan show(s) a decreasing tendency, the patient(s) correspond(s) to the moderate disease activity group.

7. The kit of claim 1, wherein the rheumatoid arthritis patient with high disease activity satisfies a DAS28-ESR score, which is the severity level of a rheumatoid arthritis patient, of 5.1 or more.

8. The kit of claim 1, wherein the rheumatoid arthritis patient with moderate disease activity satisfies a DAS28-ESR score, which is the severity level of a rheumatoid arthritis patient, of less than 5.1.

9. A kit for predicting the severity of a rheumatoid arthritis patient, comprising:
a quantification device for one or more synovial fluid metabolites selected from the group consisting of indole-3-lactate, citrate, glucose-6-phosphate, fucose, isothreonate, 3-phenyllactate, guaiacol, glycocyamine, adipate, phenylalanine, arabitol, asparagine, cholic acid, and tryptophan.

10. The kit of claim 9, wherein the quantification device is an instrument for chromatography/mass spectroscopy.

11. The kit of claim 9, wherein the concentration(s) of one or more metabolites selected from the group consisting of citrate and asparagine decrease(s) according to the increase in the severity of a rheumatoid arthritis patient.

12. The kit of claim 9, wherein the concentration(s) of one or more metabolite(s) selected from the group consisting of indole-3-lactate, glucose-6-phosphate, fucose, isothreonate, 3-phenyllactate, guaiacol, glycocyamine, adipate, phenylalanine, arabitol, cholic acid and tryptophan show(s) an increasing tendency according to the increase in the severity of a rheumatoid arthritis patient.
